# FASCICULE DE BREVET EUROPEEN

(11) **EP 0 795 323 B1**
(45) Date de publication et mention de la délivrance du brevet: **28.10.1998**
(21) Numéro de dépôt: 97400245.3
(22) Date de dépôt: 04.02.1997
(51) Int. Cl.: A61K 7/48, C08L 5/14, A61K 47/36, A61K 7/06

(54) **Composition gélifiée stable contenant des actifs lipophiles sensibles à l'oxygène et/ou à l'eau**
Stabile gelierte Zusammensetzung, die gegenüber Sauerstoff und/oder Wasser empfindliche lipophile Wirkstoffe enthält
Stable gelified composition containing oxygen- and/or water-sensitive lipophilic actives

(30) Priorité: 12.03.1996 FR 9603096
(43) Date de publication de la demande: 17.09.1997
(73) Titulaire: L'OREAL, 75008 Paris (FR)
(72) Inventeur: Gagnebien, Didier, Westfield 07090, New-Jersey (US); Pascal, Simon, 94400 Vitry-Sur-Seine (FR)
(74) Mandataire: Lhoste, Catherine

(56) Documents cités:
- EP-A- 0 281 360
- EP-A- 0 481 240
- EP-A- 0 682 936
- RESEARCH DISCLOSURE, no. 38413, 10 Avril 1996, pages 235-236, XP002033229 MAJEWICZ ET AL.: "ethyl galactomannan film properties for use in personal care applications"
- RESEARCH DISCLOSURE, no. 37807, Octobre 1995, page 642 XP002018143 MAJEWICZ ET AL.: "oil-based cosmetic and therapeutic compositions containing ethylguar"

## Description

La présente invention se rapporte à une composition gélifiée contenant, de manière stable, un actif lipophile sensible à l'oxygène et/ou à l'eau, destinée en particulier aux domaines cosmétique et/ou dermatologique. L'invention se rapporte aussi à une utilisation de cette composition pour le traitement de la peau.

Les actifs lipophiles sont connus pour leurs effets bénéfiques sur la peau, notamment en application topique. Ainsi, le rétinol est, depuis longtemps, utilisé dans le traitement de l'acné. En outre, le rétinol s'est révélé extrêmement actif dans le domaine de la correction des dommages induits soit par l'âge, soit par une exposition au soleil trop intensive.

Ainsi, les effets du rétinol sur la différenciation cellulaire permettent d'envisager, entre autre, son utilisation pour lutter efficacement contre l'apparition des rides et ridules, contre la sécheresse cutanée, la rugosité et/ou la rigidité de la peau. Son activité dans la régénération des tissus en fait un composé important dans la cicatrisation. Une application répétée de compositions cosmétiques contenant du rétinol permet, entre autre, d'effacer les rides, de lisser la peau, de réparer les petites déchirures de l'épiderme.

Du fait de ces effets bénéfiques, on cherche depuis très longtemps, à formuler le rétinol dans des compositions cosmétiquement acceptables, sous forme stable pendant au moins plusieurs mois à température ambiante.

Des compositions utilisables en cosmétique contenant du rétinol sont décrites, en particulier dans le document US-A-4 826 828 et dans le document WO-A-93/00085.

Dans le document US-A-4 826 828, il s'agit d'émulsions eau-dans-huile contenant le rétinol, une silicone volatile, ainsi qu'un solvant du rétinol et de la silicone volatile. Le solvant préféré est l'éthanol. Or, la demanderesse a montré récemment que le rétinol se dégrade en présence d'éthanol. Pour obtenir l'émulsion, il est enseigné dans ce document US-A-4 826 828 de préparer une solution contenant le rétinol à mélanger au moment de l'emploi à une émulsion eau-dans-huile pour éviter toute dégradation du rétinol. En outre, l'emploi d'antioxydant et d'agent chélateur de métaux dans la phase aqueuse est indiqué comme essentiel.

La stabilité de telles compositions, comme l'indique le détenteur du brevet US-A-4 826 828 sur les emballages de ses produits Bioadvance et Bioadvance 2000 décrits dans ce brevet, n'excède pas un mois. Ainsi, la stabilité du rétinol dans ce type de compositions est insuffisante au regard d'une utilisation prolongée, ce qui nécessite un réapprovisionnement rapide, donc coûteux.

Dans le document WO-A-93/00085, il est proposé une stabilisation du rétinol dans les compositions cosmétiques par adjonction à celles-ci d'un complexe stabilisant, comprenant associés, un antioxydant et un agent chélateur d'ions métalliques. Si, toutefois, la stabilité du rétinol semble améliorée dans de telles compositions (60 % du rétinol étant encore présent dans la composition après trois mois de conservation à 40°C), il n'en demeure pas moins que la stabilité relative du rétinol n'est due qu'à la présence, dans la composition, d'une quantité importante d'antioxydants et d'agents chélateurs stabilisants.

De nombreuses recherches ont été menées afin de diminuer au maximum, voire d'éliminer, la présence de tels stabilisants dans les compositions cosmétiques contenant du rétinol, tout en conservant à celui-ci une stabilité dans la composition, acceptable au regard de ses effets et en relation avec une utilisation prolongée de la composition.

Les problèmes d'instabilité liés au rétinol existent aussi pour ses dérivés comme le β-carotène, le palmitate de rétinol et l'acétate de rétinol, ainsi que pour les flavonoïdes et les acides gras polyinsaturés. Aussi, la présente invention s'applique à tout type d'actif sensible à l'oxygène et/ou à l'eau.

De manière surprenante et inattendue, la demanderesse a découvert qu'il était possible de formuler des compositions stables dans le temps, contenant des actifs lipophiles sensibles à l'oxygène et/ou à l'eau, destinées notamment à une utilisation topique, par un choix approprié d'un gélifiant particulier.

La présente invention a donc pour objet une composition gélifiée stable contenant au moins un actif lipophile sensible à l'oxygène et/ou à l'eau, et un solvant, caractérisée en ce qu'elle comprend, comme gélifiant, au moins un alkyléther de polysaccharide formé de motifs comportant au moins deux cycles osidiques différents, chaque motif comportant au moins un groupe hydroxyle substitué par une chaîne alkyle hydrocarbonée saturée.

Grâce à ce gélifiant particulier, la composition selon l'invention est particulièrement stable dans le temps et aucune dégradation significative de l'actif sensible à l'oxygène et/ou à l'eau et en particulier du rétinol, n'est observée.

Aussi, l'invention a encore pour objet l'utilisation d'un alkyléther de polysaccharide formé de motifs comportant au moins deux cycles osidiques différents, chaque motif comportant au moins un groupe hydroxyle substitué par une chaîne alkyle hydrocarbonée saturée, pour stabiliser un actif lipophile sensible à l'oxygène et/ou à l'eau.

Comme actif sensible à l'oxygène et/ou à l'eau, on peut citer, outre le rétinol, les dérivés du rétinol comme le β-carotène, le palmitate ou l'acétate de rétinol ; les flavonoïdes ; les acides gras polyinsaturés.

Selon un mode de réalisation particulier de l'invention, l'alkyléther de polysaccharide a un poids moléculaire supérieur à 100 000, et de préférence supérieur à 200 000. Chaque motif comporte de préférence de un à six et mieux de deux à quatre groupes hydroxyle substitués par une chaîne alkyle hydrocarbonée saturée.

Par chaîne alkyle hydrocarbonée saturée, on entend une chaîne comportant de 1 à 24, de préférence de 1 à 10 et mieux de 1 à 5 atomes de carbone. En particulier, la chaîne alkyle est choisie parmi les chaînes méthyle, éthyle, n-propyle, isopropyle, n-butyle, isobutyle, tertio-butyle, n-pentyle.

Les cycles osidiques sont notamment choisis parmi le mannose, le galactose, le glucose, le furanose, le rhamnose, l'arabinose.

Selon un mode préféré de réalisation de l'invention, l'alkyléther de polysaccharide selon l'invention est un alkyléther d'une gomme et plus particulièrement d'une gomme globalement non ionique, c'est-à-dire pratiquement sans groupe ionique. Comme gommes appropriées, on peut citer par exemple la gomme guar dont le motif comprend un galactose et un mannose, la gomme de caroube dont le motif comprend un galactose et un mannose, la gomme de karaya qui est un mélange complexe de rhamnose, galactose et acide galacturonique, la gomme adragante qui est un mélange complexe d'arabinose, galactose et acide galacturonique.

Selon un mode de réalisation préféré de l'invention, l'alkyléther de polysaccharide est un dérivé de gomme guar, et plus particulièrement la guar éthylée ayant un degré de substitution d'environ 2 à 3, en particulier 2,5, telle que décrite dans le document (RD 95378007 (octobre 1995) Research Disclosure nº 37807 p. 642 (Oct.95) et Res. Dis. nº 38413, p. 235-236 (10, avril 96).

La composition selon l'invention peut contenir par exemple une quantité d'alkyléther de polysaccharide allant de 0,5 à 10 %, et de préférence de 2 à 8 % du poids total de la composition.

L'actif lipophile sensible à l'oxygène et/ou à l'eau est présent dans la composition selon l'invention en une quantité allant de 0,001 à 20 %, de préférence de 0,01 à 5 % et mieux de 0,2 à 1 % du poids total de la composition.

Dans la composition selon l'invention, on peut utiliser n'importe quel solvant organique susceptible de dissoudre l'actif lipophile sensible à l'oxygène et/ou à l'eau tout en lui conservant une bonne stabilité. Par solvant, on entend aussi bien les solvants hydrocarbonés que les huiles. Ce solvant peut être choisi notamment parmi les alcools gras, les esters gras, les huiles d'origine végétale et/ou leurs mélanges, ainsi que les mélanges de ces solvants avec des huiles siliconées et/ou minérales. En particulier, ce solvant est choisi dans le groupe constitué par les alcools gras aliphatiques à chaine droite ou ramifiée en C₁₆-C₂₀, les diesters d'acide dicarboxylique en C₆-C₁₄ et d'alcool isopropylique, les triglycérides d'acide gras en C₆-C₁₈ et leurs mélanges.

Parmi les alcools gras aliphatiques en C₁₆-C₂₀ utilisables dans l'invention, on peut citer l'hexyl-2-décanol tel que celui vendu par la Société Condea sous la dénomination "Isofol 16", l'octyldodécanol tel que celui vendu par la Société Henkel sous la dénomination "Eutanol G", et l'alcool isostéarylique tel que celui vendu par la Société Sherex sous la dénomination de "Adol 66".

Parmi les diesters dicarboxyliques en C₆-C₁₄ et d'alcool isopropylique utilisables dans l'invention, on peut citer l'adipate de di-isopropyle tel que celui vendu par la Société ISP sous la dénomination de "Ceraphyl 230".

Parmi les triglycérides d'acides gras en C₆-C₁₈, selon l'invention, on préfère utiliser des triglycérides mixtes des acides capriques/capryliques tels que ceux vendus par la Société Hüls France sous la dénomination de "Miglyol 812".

Dans une réalisation particulière de l'invention, il est possible d'utiliser le mélange de deux ou plusieurs de ces solvants de l'actif lipophile sensible à l'oxygène et/ou à l'eau.

La composition selon l'invention peut de plus comprendre tous les ingrédients classiquement utilisés dans le domaine cosmétique ou dermatologique, dans des concentrations habituelles. Ces ingrédients sont en particuliers choisis parmi les corps gras, les conservateurs, les vitamines et autres actifs, les gélifiants, les parfums, les tensioactifs, l'eau, les antioxydants, les charges, les filtres, les hydratants et leurs mélanges. La composition selon l'invention peut contenir également des vésicules lipidiques de type ionique et/ou non ionique. Les quantités de ces différents adjuvants sont celles classiquement utilisées dans les domaines cosmétique et/ou dermatologique, et par exemple de 0,01 % à 20 % du poids total de la composition.

D'autres constituants conventionnels des compositions cosmétiques, pharmaceutiques ou vétérinaires peuvent être introduits dans les émulsions selon l'invention. La nature de ces ingrédients et leur proportion doit être compatible avec la stabilité recherchée de l'actif sensible à l'oxygène et/ou à l'eau dans les compositions selon l'invention.

La composition de l'invention peut se présenter sous toutes les formes galéniques normalement utilisées pour une application topique, notamment sous forme d'un gel huileux, d'une émulsion huile-dans-eau ou eau-dans-huile. Cette composition peut être plus ou moins fluide et avoir l'aspect d'une crème, d'une pommade, d'une pâte, d'une mousse.

Lorsque la composition de l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 à 30 % en poids, et de préférence de 0,5 à 20 % en poids par rapport au poids total de la composition. L'émulsion peut, en outre, contenir des vésicules lipidiques.

Comme émulsionnants utilisables dans l'invention, on peut citer par exemple les émulsionnants siliconés, notamment les alkyl diméthicone copolyols en C₁₀-C₂₂ polyoxyéthylénés tels que le cétyl diméthicone copolyol ou "Abil EM-90" de la Société Goldschmidt, ou le lauryl diméthicone copolyol polyoxyéthyléné et polyoxypropyléné comme le Q2-5200 de Dow Corning, et un mélange des deux.

On peut aussi utiliser les diméthicone copolyols polyoxyéthylénés tel que le SP 1228 de General Electric et également le Q2-3225 C de Dow Corning.

La phase huileuse de l'émulsion peut également contenir une huile complémentaire inerte vis-à-vis de l'actif sensible à l'oxygène et/ou à l'eau, choisie de préférence parmi les huiles minérales ou siliconées.

Parmi les huiles minérales utilisables selon l'invention, on peut citer, entre autre, l'isohexadécane, la paraffine, l'isoparaffine, la vaseline.

Parmi les huiles siliconées utilisables dans l'invention, on peut citer, entre autre, les diméthicones, les diméthiconols, les cyclométhicones telles que la cyclopentadiméthylsiloxane (ou cyclométhicone D5) ou la cyclohexadiméthylsiloxane (ou cyclométhicone D6) ou les alkyl diméthicones et un mélange de certains de ces composés tel que la "gomme Q2-1401" de Dow Corning.

La composition selon l'invention peut être notamment utilisée pour le traitement de la peau, en particulier pour traiter l'acné et les dommages induits par l'âge et/ou le soleil, et plus spécialement les rides et/ou les ridules et/ou le vieillissement de la peau. Par conséquent, l'invention a encore pour objet l'utilisation cosmétique et/ou dermatologique de la composition telle que définie ci-dessus pour traiter l'acné et/ou les rides et/ou les ridules et/ou le vieillissement de la peau.

Les exemples ci-après de compositions selon l'invention, sont des exemples hypothétiques réalisables donnés à titre d'illustration et sans caractère limitatif. Les quantités y sont données en % en poids.

### Exemple 1 :

| | |
|---|---|
| Rétinol | 0,15 % |
| Guar éthylée ayant un degré de substitution d'environ 2,5 | 4 % |
| Hexyl-2-décanol (Isofol-16) | qsp 100 % |

Ce gel pourrait être utilisé notamment pour le traitement de l'acné.

### Exemple 2 :

Cet exemple se différencie de l'exemple 1 par l'utilisation du Miglyol 812 à la place de l'Isofol-16.

### Exemple 3 :

| *Phase A :* | |
|---|---|
| Abil EM-90 | 2 % |
| Cyclométhicone D5 | 9,5 % |
| Miglyol 812 | 10 % |
| Gomme Q2 1401 | 4 % |

| *Phase B:* | |
|---|---|
| Glycérine | 5 % |
| Triéthanolamine | 0,2 % |
| Eau permutée | 61,3 % |

| *Phase C:* | |
|---|---|
| Isofol 16 | 7,5 % |
| Guar éthylée ayant un degré de substitution d'environ 2,5 | 2 % |
| Rétinol | 0,5 % |

Cette crème appliquée en contour des yeux en cure de 2 à 5 fois par semaine devrait avoir un effet de lissage des fines ridules du contour de l'oeil.

### Exemple 4 :

Cet exemple se différencie de l'exemple 1 par l'utilisation du palmitate de rétinol au lieu du rétinol.

## Revendications

1. Composition gélifiée stable contenant au moins un actif lipophile sensible à l'oxygène et/ou à l'eau, et un solvant, caractérisée en ce qu'elle comprend, comme gélifiant, au moins un alkyléther de polysaccharide formé de motifs comportant au moins deux cycles osidiques différents, chaque motif comportant au moins un groupe hydroxyle substitué par une chaîne alkyle hydrocarbonée saturée.

2. Composition selon la revendication 1, caractérisée en ce que chaque motif comporte deux à quatre groupes hydroxyle substitués par une chaîne alkyle hydrocarbonée saturée.

3. Composition selon la revendication 1 ou 2, caractérisée en ce que la chaîne alkyle hydrocarbonée saturée comporte de 1 à 24 atomes de carbone.

4. Composition selon l'une quelconque des revendications précédentes, caractérisée en ce que la chaîne alkyle hydrocarbonée saturée comporte de 1 à 5 atomes de carbone.

5. Composition selon l'une des revendications précédentes, caractérisée en ce que la chaîne alkyle est choisie parmi les chaînes méthyle, éthyle, n-propyle, isopropyle, n-butyle, isobutyle, tertiobutyle, pentyle.

6. Composition selon l'une quelconque des revendications précédentes, caractérisée en ce que les cycles osidiques sont choisis parmi le mannose, le galactose, le glucose, le furanose, le rhamnose, l'arabinose.

7. Composition selon l'une quelconque des revendications précédentes, caractérisée en ce que l'alkyléther de polysaccharide est un alkyléther d'une gomme choisie parmi la gomme guar, la gomme de caroube, la gomme de karaya, la gomme adragante et leurs mélanges.

8. Composition selon l'une quelconque des revendications précédentes, caractérisée en ce que l'alkyléther de polysaccharide a un poids moléculaire supérieur à 200 000.

9. Composition selon l'une quelconque des revendications précédentes, caractérisée en ce que l'alkyléther de polysaccharide est présent en une quantité allant de 0,5 à 10 % du poids total de la composition.

10. Composition selon l'une quelconque des revendications précédentes, caractérisée en ce que l'alkyléther de polysaccharide est présent en une quantité allant de 2 à 8 % du poids total de la composition.

11. Composition selon l'une quelconque des revendications précédentes, caractérisée en ce que l'actif lipophile sensible à l'oxygène et/ou à l'eau est choisi parmi le rétinol et ses dérivés, les flavonoïdes et les acides gras polyinsaturés.

12. Composition selon l'une quelconque des revendications précédentes, caractérisée en ce que l'actif lipophile sensible à l'oxygène et/ou à l'eau est présent en une quantité allant de 0,01 à 5 % du poids total de la composition.

13. Composition selon l'une quelconque des revendications précédentes, caractérisée en ce que le solvant est choisi parmi les alcools gras, les esters gras, les huiles végétales et/ou leurs mélanges, ainsi que les mélanges de ces solvants avec des huiles siliconées et/ou minérales.

14. Composition selon l'une quelconque des revendications précédentes, caractérisée en ce qu'elle est un gel huileux, une émulsion eau-dans-huile ou une émulsion huile-dans-eau.

15. Utilisation d'un alkyléther de polysaccharide formé de motifs comportant au moins deux cycles osidiques différents, chaque motif comportant au moins un groupe hydroxyle substitué par une chaîne alkyle hydrocarbonée saturée, pour stabiliser un actif lipophile sensible à l'oxygène et/ou à l'eau.

16. Utilisation selon la revendication précédente, caractérisée en ce que l'alkyléther de polysaccharide est un alkyléther d'une gomme choisie parmi la gomme guar, la gomme de caroube, la gomme de karaya, la gomme adragante et leurs mélanges.

17. Utilisation cosmétique de la composition selon les revendications 1 à 14 pour traiter l'acné et/ou les rides et/ou les ridules et/ou le vieillissement de la peau.

## Claims

1. Stable gelled composition containing at least one lipophilic active agent sensitive to oxygen and/or to water, and a solvent, characterized in that it comprises, as gelling agent, at least one polysaccharide alkyl ether formed of units comprising at least two different glycoside rings, each unit comprising at least one hydroxyl group substituted with a saturated hydrocarbon alkyl chain.

2. Composition according to Claim 1, characterized in that each unit comprises two to four hydroxyl groups substituted with a saturated hydrocarbon alkyl chain.

3. Composition according to Claim 1 or 2, characterized in that the saturated hydrocarbon alkyl chain comprises from 1 to 24 carbon atoms.

4. Composition according to any one of the preceding claims, characterized in that the saturated hydrocarbon alkyl chain comprises from 1 to 5 carbon atoms.

5. Composition according to one of the preceding claims, characterized in that the alkyl chain is chosen from methyl, ethyl, n-propyl, isopropyl, n-butyl, isobutyl, tert-butyl and pentyl chains.

6. Composition according to any one of the preceding claims, characterized in that the glycoside rings are chosen from mannose, galactose, glucose, furanose, rhamnose and arabinose.

7. Composition according to any one of the preceding claims, characterized in that the polysaccharide alkyl ether is an alkyl ether of a gum chosen from guar gum, carob gum, karaya gum, tragacanth gum and mixtures thereof.

8. Composition according to any one of the preceding claims, characterized in that the polysaccharide alkyl ether has a molecular weight greater than 200,000.

9. Composition according to any one of the preceding claims, characterized in that the polysaccharide alkyl ether is present in a quantity ranging from 0.5 to 10% of the total weight of the composition.

10. Composition according to any one of the preceding claims, characterized in that the polysaccharide alkyl ether is present in a quantity ranging from 2 to 8% of the total weight of the composition.

11. Composition according to any one of the preceding claims, characterized in that the lipophilic active agent sensitive to oxygen and/or to water is chosen from retinol and its derivatives, flavonoids and polyunsaturated fatty acids.

12. Composition according to any one of the preceding claims, characterized in that the lipophilic active agent sensitive to oxygen and/or to water is present in a quantity ranging from 0.01 to 5% of the total weight of the composition.

13. Composition according to any one of the preceding claims, characterized in that the solvent is chosen from fatty alcohols, fatty esters, vegetable oils and/or mixtures thereof, as well as mixtures of these solvents with silicone and/or mineral oils.

14. Composition according to any one of the preceding claims, characterized in that it is an oily gel, a water-in-oil emulsion or an oil-in-water emulsion.

15. Use of a polysaccharide alkyl ether formed of units comprising at least two different glycoside rings, each unit comprising at least one hydroxyl group substituted with a saturated hydrocarbon alkyl chain, to stabilize a lipophilic active agent sensitive to oxygen and/or to water.

16. Use according to the preceding claim, characterized in that the polysaccharide alkyl ether is an alkyl ether of a gum chosen from guar gum, carob gum, karaya gum, tragacanth gum and mixtures thereof.

17. Cosmetic use of the composition according to Claims 1 to 14 to treat acne and/or normal and/or small wrinkles and/or ageing of the skin.

## Patentansprüche

1. Stabile gelierte Zusammensetzung, die mindestens einen gegenüber Sauerstoff und/oder Wasser empfindlichen lipophilen Wirkstoff und ein Lösungsmittel enthält, dadurch gekennzeichnet, daß sie als Gelbildner mindestens einen Polysaccharidalkylether enthält, der aus Einheiten gebildet ist, die mindestens zwei verschiedene Zuckerringe enthalten, wobei jede Einheit mindestens eine Hydroxygruppe aufweist, die mit einer gesättigten Alkylgruppe substituiert ist.

2. Zusammensetzung nach Anspruch 1, dadurch gekennzeichnet, daß jede Einheit zwei bis vier Hydroxygruppen aufweist, die mit einer gesättigten Alkylgruppa substituiert sind.

3. Zusammensetzung nach Anspruch 1 oder 2, dadurch gekennzeichnet, daß die gesättigte Alkylgruppe 1 bis 24 Kohlenstoffatome aufweist.

4. Zusammensetzung nach einem der vorhergehenden Ansprüche, dadurch gekennzeichnet, daß die gesättigte Alkylgruppe 1 bis 5 Kohlenstoffatome aufweist.

5. Zusammensetzung nach einem der vorhergehenden Ansprüche, dadurch gekennzeichnet, daß die Alkylgruppe unter Methyl, Ethyl, n-Propyl, Isopropyl, n-Butyl, Isobutyl, tert.-Butyl und Pentyl ausgewählt ist.

6. Zusammensetzung nach einem der vorhergehenden Ansprüche, dadurch gekennzeichnet, daß die Zuckerringe unter Mannose, Galactose, Glucose, Furanose, Rhamnose und Arabinose ausgewählt sind.

7. Zusammensetzung nach einem der vorhergehenden Ansprüche, dadurch gekennzeichnet, daß es sich bei dem Polysaccharidalkylether um einen Alkylether eines Gummis handelt, das unter Guargummi, Carubin, Karaya-Gummi, Tragant und deren Gemischen ausgewählt ist.

8. Zusammensetzung nach einem der vorhergehenden Ansprüche, dadurch gekennzeichnet, daß der Polysaccharidalkylether ein Molekulargewicht von über 200 000 aufweist.

9. Zusammensetzung nach einem der vorhergehenden Ansprüche, dadurch gekennzeichnet, daß der Polysaccharidalkylether in einem Mengenanteil von 0,5 bis 10 % des Gesamtgewichts der Zusammensetzung vorliegt.

10. Zusammensetzung nach einem der vorhergehenden Ansprüche, dadurch gekennzeichnet, daß der Polysaccharidalkylether in einem Mengenanteil von 2 bis 8 % das Gesamtgewichts der Zusammensetzung vorliegt.

11. Zusammensetzung nach einem der vorhergehenden Ansprüche, dadurch gekennzeichnet, daß der gegenüber Sauerstoff und/oder Wasser empfindliche lipophile Wirkstoff unter Retinol und seinen Derivaten, Flavonoiden und mehrfach ungesättigten Fettsäuren ausgewählt ist.

12. Zusammensetzung nach einem der vorhergehenden Ansprüche, dadurch gekennzeichnet, daß der gegenüber Sauerstoff und/oder Wasser empfindliche lipophile Wirkstoff in einem Mengenanteil von 0,01 bis 5 % des Gesamtgewichts der Zusammensetzung vorliegt.

13. Zusammensetzung nach einem der vorhergehenden Ansprüche, dadurch gekennzeichnet, daß das Lösungsmittel unter den Fettalkoholen, Fettestern, pflanzlichen Ölen und/oder deren Gemischen sowie den Gemischen dieser Lösungsmittel mit Siliconölen und/oder Mineralölen ausgewählt ist.

14. Zusammensetzung nach einem der vorhergehenden Ansprüche, dadurch gekennzeichnet, daß sie ein öliges Gel, eine Wasser-in-Öl-Emulsion oder eine Öl-in-Wasser-Emulsion ist.

15. Verwendung eines Polysaccharidalkylethers, der aus Einheiten gebildet ist, die mindestens zwei verschiedene Zuckerringe enthalten, wobei jede Einheit mindestens eine Hydroxygruppe aufweist, die mit einer gesättigten Alkylgruppe substituiert ist, zur Stabilisierung eines gegenüber Sauerstoff und/oder Wasser empfindlichen lipophilen Wirkstoffs.

16. Verwendung nach dem vorhergehenden Anspruch, dadurch gekennzeichnet, daR es sich bei dem Polysaccharidalkylether um einen Alkylether eines Gummis handelt, das unter Guargummi, Carubin, Karaya-Gummi, Tragant und deren Gemischen ausgewählt ist.

17. Kosmetische Verwendung der Zusammensetzung nach den Ansprüchen 1 bis 14 zur Behandlung von Akne und/oder Falten und/oder Fältchen und/oder der Hautalterung.
